# EUROPEAN PATENT SPECIFICATION

(11) **EP 4 546 573 B1**
(45) Date of publication and mention of the grant of the patent: **18.02.2026**
(21) Application number: 24199328.6
(22) Date of filing: 09.09.2024
(51) Int. Cl.: H01R 13/00, A61B 5/256, A61B 5/271, H01R 12/63, H01R 12/70, A61B 5/00

(54) **CONNECTOR ASSEMBLY AND CONNECTOR**
VERBINDERANORDNUNG UND VERBINDER
ENSEMBLE CONNECTEUR ET CONNECTEUR

(30) Priority: 26.10.2023 JP 2023183832
(43) Date of publication of application: 30.04.2025
(73) Proprietor: Japan Aviation Electronics Industry, Limited, Tokyo 150-0043 (JP)
(72) Inventor: KOMOTO, Tetsuya, Tokyo, 150-0043 (JP)
(74) Representative: Qip Patent & Recht Dr. Kuehn & Partner mbB

(56) References cited:
- WO-A1-2016/132879
- US-A1- 2018 325 396
- US-A1- 2020 022 425

## Description

### BACKGROUND OF THE INVENTION

The present invention relates to a connector configured to be mounted on a flexible mounting object and a connector assembly.

In recent years, attention has been drawn to so-called smart clothes that can obtain user's biological data such as the heart rate and the body temperature only by being worn by the user. Such smart clothes have an electrode disposed at a measurement site, and when a wearable device serving as a measurement device is electrically connected to the electrode, biological data can be transmitted to the wearable device.

The electrode and the wearable device can be interconnected by, for instance, use of a connector for garments that is mounted on a garment as smart clothes.

Moreover, for instance, when electrocardiogram, blood pressure or the like is measured, for the sake of reducing the user's load, it is desired to blow air onto a user's body surface under compression by an electrode. However, the connector for garments as described above has difficulty in distributing a fluid such as air although the connector is capable of electric connection.

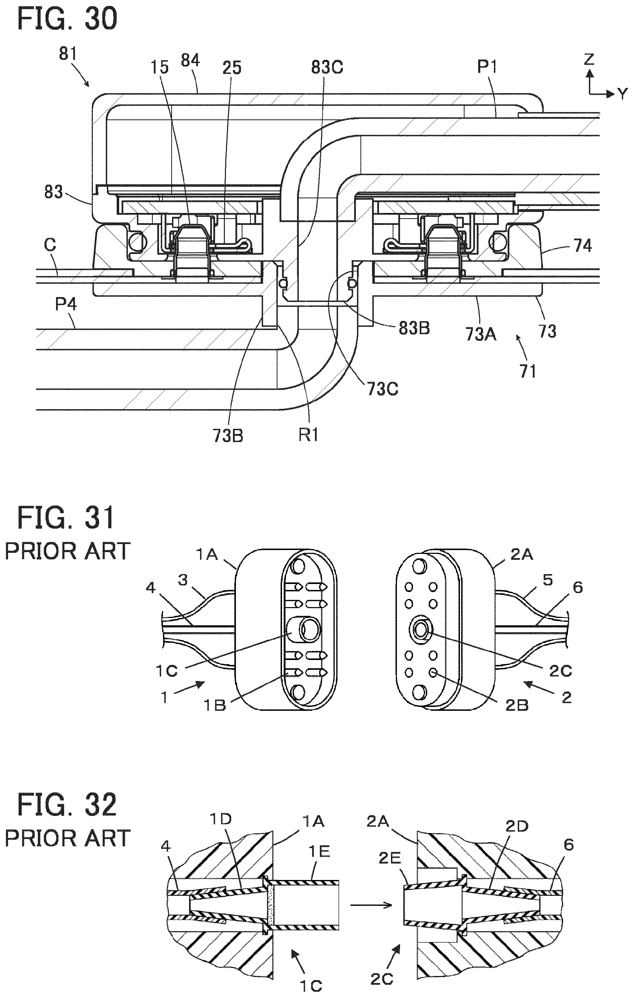

For example, WO 2016/132879 discloses a connecting device that performs connection of a fluid passage for distributing air, liquids, or the like and electric connection between a subject and a measurement apparatus at a time. The connecting device includes a living body-side connector 1 and a measurement apparatus-side connector 2 as shown in FIG. 31.

The living body-side connector 1 includes a housing 1A, a plurality of connection terminals 1B held by the housing 1A and attached to an end portion of a living body-side cable 3, and a connection port 1C held by the housing 1A and connected to an end portion of a living body-side tube 4. Meanwhile, the measurement apparatus-side connector 2 includes a housing 2A, a plurality of connection terminals 2B held by the housing 2A and attached to an end portion of the measurement apparatus-side cable 5, and a connection port 2C held by the housing 2A and connected to an end portion of a measurement apparatus-side tube 6.

As shown in FIG. 32, the connection port 1C of the living body-side connector 1 is composed of a rear end-side nozzle portion 1D joined to the end portion of the living body-side tube 4 and a tip end-side nozzle portion 1E joined to the rear end-side nozzle portion 1D with a tube joint and attached to the housing 1A, and the connection port 2C of the measurement apparatus-side connector 2 is composed of a rear end-side nozzle portion 2D joined to the end portion of the measurement apparatus-side tube 6 and a tip end-side nozzle portion 2E joined to the rear end-side nozzle portion 2D with a tube joint and attached to the housing 2A.

By use of such a connecting device, electric connection between the living body-side cable 3 and the measurement apparatus-side cable 5 and connection of a fluid passage between the living body-side tube 4 and the measurement apparatus-side tube 6 can be made at a time.

However, it is difficult to mount the connecting device of WO 2016/132879 on a flexible mounting object such as garments, disadvantageously.

In addition, for producing the connection port 1C of the living body-side connector 1, it is necessary to attach the tip end-side nozzle portion 1E to the housing 1A and join the rear end-side nozzle portion 1D to the tip end-side nozzle portion 1E with a tube joint, and similarly, for producing the connection port 2C of the measurement apparatus-side connector 2, it is necessary to attach the tip end-side nozzle portion 2E to the housing 2A and join the rear end-side nozzle portion 2D to the tip end-side nozzle portion 2E with a tube joint, resulting in complicated production process of the connecting device, disadvantageously.

US 2018/0325396 A1 discloses a finger cuff connector for a blood pressure measurement system.

US 2020/0022425 A1 discloses a garment connector.

### SUMMARY OF THE INVENTION

The present invention has been made in order to solve the conventional problems described above and aims at providing a connector assembly and a connector that can be mounted on a flexible mounting object and can perform electric connection and connection of a fluid passage at a time while easy production thereof is possible.

The connector according to the present invention is a connector as claimed in claim 1.

The connector assembly according to the present invention is a connector assembly as claimed in claim 2.

### BRIEF DESCRIPTION OF THE DRAWINGS

FIG. 1 is a perspective view showing a connector assembly according to Embodiment 1 in the non-fitted state, when viewed from an obliquely upper position.
FIG. 2 is a perspective view showing the connector assembly according to Embodiment 1 in the non-fitted state, when viewed from an obliquely lower position.
FIG. 3 is an assembly view of a first connector in Embodiment 1.
FIG. 4 is a perspective view showing a first bottom insulator used in the first connector in Embodiment 1.
FIG. 5 is a perspective view showing a first top insulator used in the first connector in Embodiment 1.
FIG. 6 is a perspective view showing a reinforcement sheet used in the first connector in Embodiment 1.
FIG. 7 is a perspective view showing a first contact member used in the first connector in Embodiment 1.
FIG. 8 is a cross-sectional view showing the first contact member used in the first connector in Embodiment 1.
FIG. 9 is a perspective view showing a mounting object on which the first connector is mounted in Embodiment 1.
FIG. 10 is a cross-sectional view showing the first connector in Embodiment 1 which is mounted on the mounting object and to which a mounting object-side distribution tube is attached.
FIG. 11 is an assembly view of a second connector in Embodiment 1.
FIG. 12 is a perspective view showing a second bottom insulator used in the second connector in Embodiment 1.
FIG. 13 is a perspective view showing a second top insulator used in the second connector in Embodiment 1.
FIG. 14 is a perspective view showing a second contact member used in the second connector in Embodiment 1.
FIG. 15 is a perspective view showing a circuit board used in the second connector in Embodiment 1.
FIG. 16 is a perspective view showing a **fluid** distribution tube to which the second connector is attached in Embodiment 1.
FIG. 17 is a cross-sectional view showing the second connector in Embodiment 1 attached to the **fluid** distribution tube.
FIG. 18 is a perspective view showing the connector assembly according to Embodiment 1 in the fitted state.
FIG. 19 is a cross-sectional view showing the connector assembly according to Embodiment 1 in the fitted state.
FIG. 20 is a cross-sectional view showing a first connector in Modification 1 of Embodiment 1.
FIG. 21 is a cross-sectional view showing a first connector in Modification 2 of Embodiment 1.
FIG. 22 is a cross-sectional view showing a first connector in Modification 3 of Embodiment 1.
FIG. 23 is an assembly view of a second connector in a connector assembly of Embodiment 2.
FIG. 24 is a perspective view showing a second bottom insulator used in the second connector in Embodiment 2.
FIG. 25 is a perspective view showing a **fluid** distribution tube to which the second connector is attached in Embodiment 2.
FIG. 26 is a cross-sectional view showing the second connector in Embodiment 2 attached to the **fluid** distribution tube.
FIG. 27 is a perspective view showing a connector assembly according to Embodiment 3 in the non-fitted state, when viewed from an obliquely upper position.
FIG. 28 is a perspective view showing the connector assembly according to Embodiment 3 in the non-fitted state, when viewed from an obliquely lower position.
FIG. 29 is a perspective view showing the connector assembly according to Embodiment 3 in the fitted state.
FIG. 30 is a cross-sectional view showing the connector assembly according to Embodiment 3 in the fitted state.
FIG. 31 is a perspective view showing a living body-side connector and a measurement apparatus-side connector in a conventional connecting device.
FIG. 32 is a cross-sectional view showing the structure of connection ports of the living body-side connector and the measurement apparatus-side connector in the conventional connecting device.

### DETAILED DESCRIPTION OF THE INVENTION

Embodiments of the present invention are described below based on the accompanying drawings.

### [Embodiment 1]

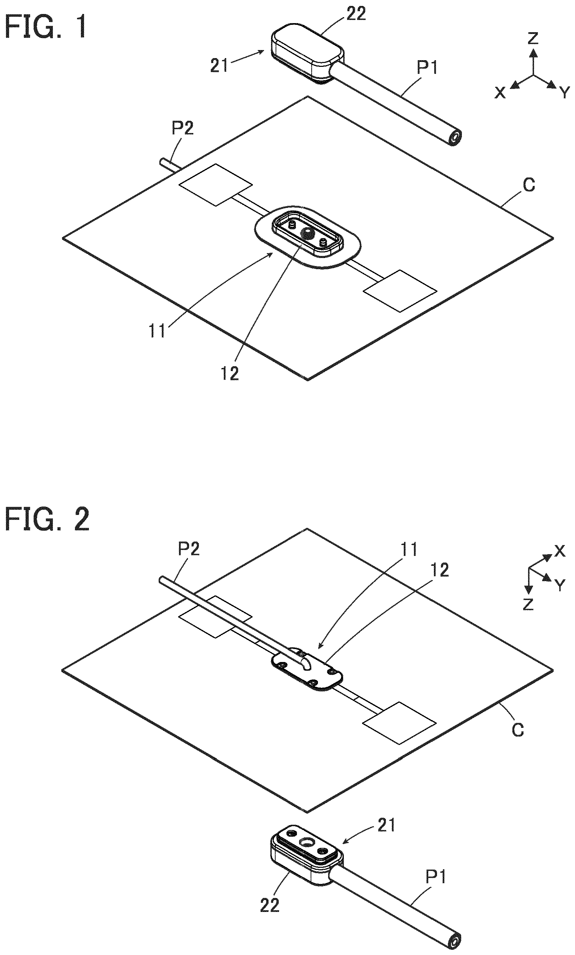

FIGS. 1 and 2 show a connector assembly according to Embodiment 1 in the non-fitted state. The connector assembly is composed of a first connector (connector) 11 and a second connector (counter connector) 21 to be fitted to each other along a fitting direction. The first connector 11 includes a first housing (housing) 12 mounted on a flexible mounting object C such as fabric of a garment, for example, and the second connector 21 includes a second housing 22 attached to a tip of a fluid distribution tube P1.

For convenience, the mounting object C is defined as extending along an XY plane, the direction in which the fluid distribution tube P1 extends is referred to as "Y direction," and the direction from the first connector 11 to the second connector 21 is referred to as "+Z direction." The Z direction is the fitting direction in which the first connector 11 and the second connector 21 are fitted together.

The second connector 21 is disposed on the +Z direction side of the mounting object C, and a mounting object-side distribution tube P2 is joined to the first housing 12 of the first connector 11 on the -Z direction side of the mounting object C.

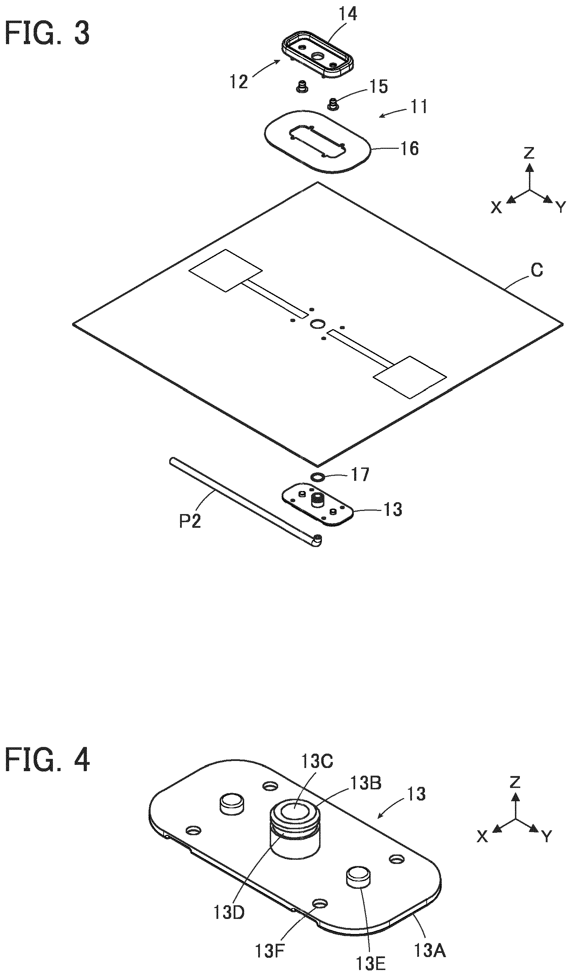

FIG. 3 shows an assembly view of the first connector 11. The first housing 12 is made of an insulating resin and is formed of a first bottom insulator 13 disposed on the - Z direction side of the mounting object C, and a first top insulator 14 disposed on the +Z direction side of the mounting object C.

A pair of first contact members (contact members) 15 are disposed on the -Z direction side of the first top insulator 14, a reinforcement sheet 16 is disposed on the -Z direction side of the pair of first contact members 15, and the mounting object C is disposed on the -Z direction side of the reinforcement sheet 16. Furthermore, the first bottom insulator 13 is disposed on the -Z direction side of the mounting object C via an O-ring 17, and the mounting object-side distribution tube P2 is disposed on the -Z direction side of the first bottom insulator 13.

As shown in FIG. 4, the first bottom insulator 13 includes a flat plate portion 13A extending along an XY plane, and, at a center portion of the flat plate portion 13A, a passage connection port 13B of cylindrical shape is integrally formed to extend from the +Z directional surface of the flat plate portion 13A in the +Z direction. The passage connection port 13B is provided in its interior with a linking passage 13C extending in the Z direction and penetrating the first bottom insulator 13. Although not shown in FIG. 4, the linking passage 13C penetrates the first bottom insulator 13, whereby an opening end portion of the linking passage 13C constitutes a first distribution port R1 opening toward an outside of the first connector 11 in the -Z directional surface of the flat plate portion 13A.

At an outer peripheral portion of the passage connection port 13B, an annual groove 13D is formed along an XY plane to surround the passage connection port 13B.

The +Z directional surface of the flat plate portion 13A is provided with a pair of projections 13E projecting in the +Z direction and formed on opposite sides of the passage connection port 13B in the Y direction across the passage connection port 13B.

In addition, a plurality of through-holes 13F are formed in the vicinity of a peripheral edge portion of the flat plate portion 13A.

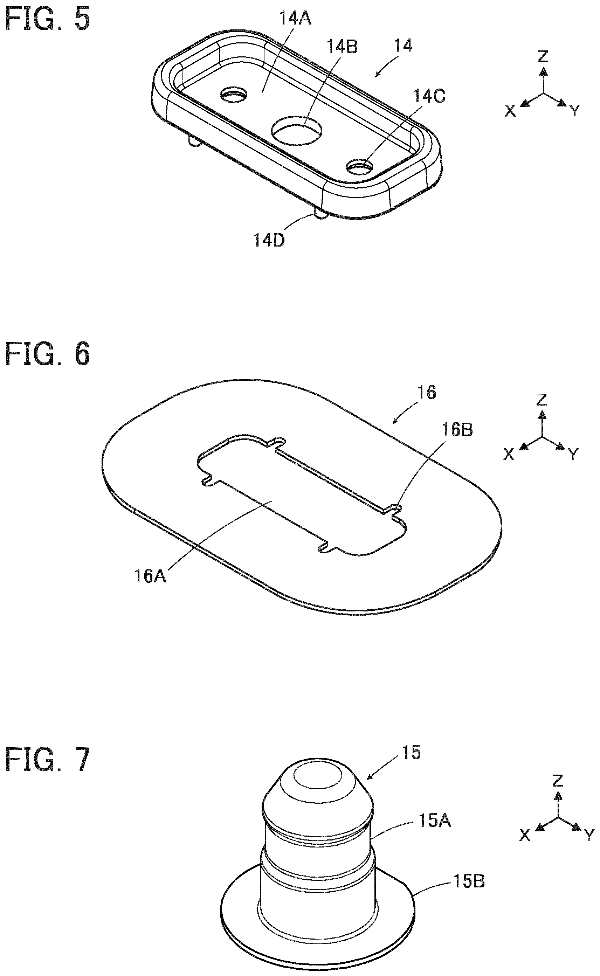

As shown in FIG. 5, the first top insulator 14 includes a recessed second connector accommodation portion 14A opening in the +Z direction, and, at a center portion of the second connector accommodation portion 14A, a connection port through-hole 14B is formed to penetrate the first top insulator 14 in the Z direction. In the second connector accommodation portion 14A, a pair of contact through-holes 14C are also formed on opposite sides of the connection port through hole 14B in the Y direction across the connection port through-hole 14B to penetrate the first top insulator 14 in the Z direction.

In addition, at a peripheral portion of a surface, facing in the -Z direction, of the first top insulator 14, a plurality of bosses 14D are formed to project in the -Z direction. The plurality of bosses 14D separately correspond to the plurality of through-holes 13F of the first bottom insulator 13.

As shown in FIG. 6, the reinforcement sheet 16 is used to reinforce the mounting object C on which the first connector 11 is mounted. The reinforcement sheet 16 is made of an insulating material and provided at its center with an opening portion 16A. Further, a plurality of cutouts 16B separately corresponding to the plurality of bosses 14D of the first top insulator 14 are formed along the periphery of the opening portion 16A of the reinforcement sheet 16.

As shown in FIG. 7, the first contact member 15 is made of an electrically conductive material such as metal and includes: a tubular portion 15A of cylindrical shape projecting in the +Z direction; and a flange 15B of disc shape extending in a radial direction along an XY plane from the outer periphery of a -Z directional end portion of the tubular portion 15A.

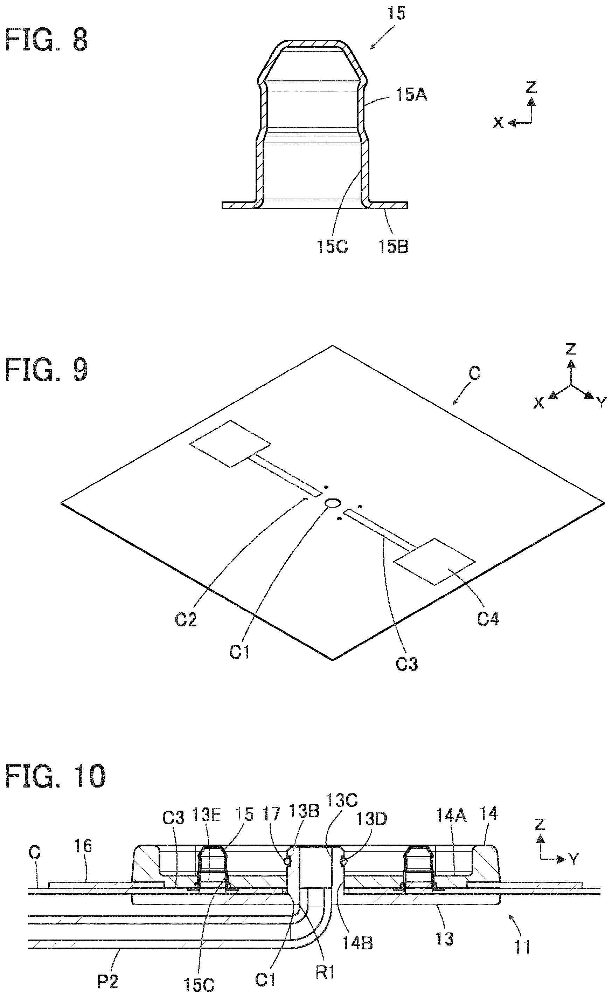

As shown in FIG. 8, the tubular portion 15A is provided in its interior with a recessed portion 15C opening in the -Z direction.

As shown in FIG. 9, the mounting object C is formed of, for example, fabric of a garment, and is provided with a single through-hole C1 through which the passage connection port 13B of the first bottom insulator 13 passes, and a plurality of through-holes C2 disposed around the through-hole C1 and corresponding to the plurality of bosses 14D of the first top insulator 14.

The mounting object C is also provided with a pair of wiring portions C3 and a pair of electrodes C4 formed of, for example, embroidery using embroidery threads made of a conductive thread. The pair of wiring portions C3 separately extend in the +Y direction and the -Y direction form the vicinity of the through-hole C1 and are connected to the corresponding electrodes C4.

When the first connector 11 is assembled, first, the tubular portion 15A of the first contact member 15 is inserted to the corresponding one of the pair of contact through-holes 14C of the first top insulator 14 from the -Z direction, and the plurality of bosses 14D of the first top insulator 14 are sequentially inserted to the plurality of cutouts 16B of the reinforcement sheet 16 and the plurality of through-holes C2 of the mounting object C. In this process, the pair of first contact members 15 are situated within the opening portion 16A of the reinforcement sheet 16, and the connection port through-hole 14B of the first top insulator 14 is situated at a position overlapping the through-hole C1 of the mounting object C on the +Z direction side.

Subsequently, the O-ring 17 is fitted into the annular groove 13D of the passage connection port 13B of the first bottom insulator 13, and the first bottom insulator 13 is pressed from the -Z direction side of the mounting object C toward the first top insulator 14, with the mounting object C being held therebetween. In this process, as shown in FIG. 10, the passage connection port 13B of the first bottom insulator 13 passes through the through-hole C1 of the mounting object C and the connection port through-hole 14B of the first top insulator 14 to project in the second connector accommodation portion 14A of the first top insulator 14, and the pair of projections 13E of the first bottom insulator 13 are separately inserted into the recessed portions 15C of the corresponding first contact members 15 while pushing the pair of wiring portions C3 of the mounting object C therein.

Accordingly, the wiring portion C3 of the mounting object C pushed into the recessed portion 15C of the first contact member 15 is held between a lateral surface of the projection 13E of the first bottom insulator 13 and an inner surface of the recessed portion 15C of the first contact member 15 and is electrically connected to the first contact member 15.

In addition, by pressing the first bottom insulator 13 toward the first top insulator 14, the bosses 14D of the first top insulator 14 sequentially passing through the cutouts 16B of the reinforcement sheet 16 and the through-holes C2 of the mounting object C pass through the through-holes 13F of the first bottom insulator 13. Thereafter, tips of the bosses 14D projecting on the -Z direction side of the first bottom insulator 13 are thermally deformed, whereby the first bottom insulator 13 and the first top insulator 14 are fixed to each other, and the assembling of the first connector 11 is completed while the first connector 11 is mounted on the mounting object C.

In order to reinforce the mounting object C, the reinforcement sheet 16 extending to the outside of the first bottom insulator 13 and the first top insulator 14 is preferably fixed to the mounting object C by sewing or other means.

Moreover, as shown in FIG. 10, the end portion of the mounting object side-distribution tube P2 is inserted from the -Z direction into the linking passage 13C formed in the interior of the passage connection port 13B of the first bottom insulator 13, whereby the mounting object-side distribution tube P2 is joined to the first distribution port R1 that is formed of the opening end portion on the -Z direction side of the linking passage 13C and is opening toward the outside of the first connector 11.

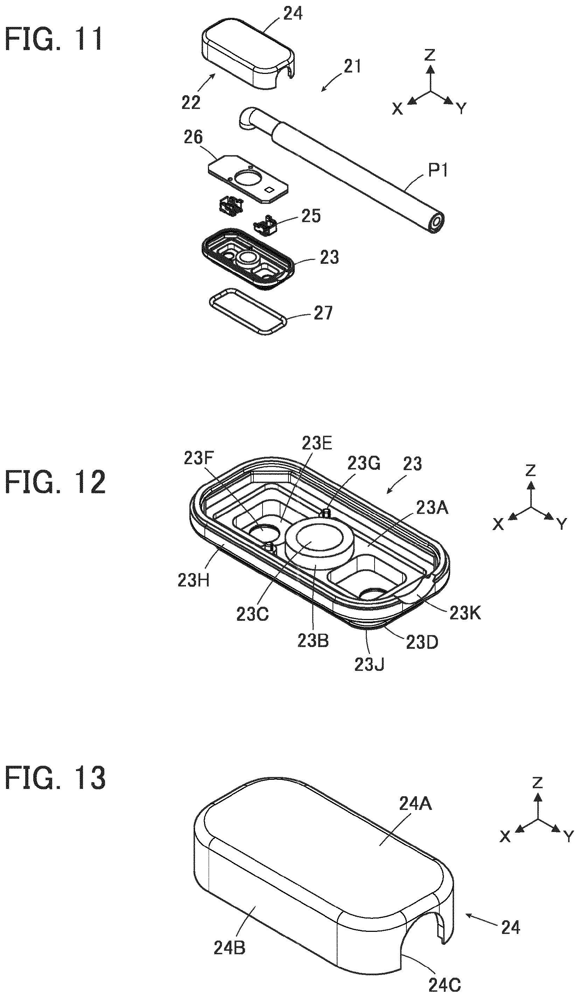

FIG. 11 shows an assembly view of the second connector 21. The second housing 22 is made of an insulating resin and is formed of a second bottom insulator 23 and a second top insulator 24.

The fluid distribution tube P1 is disposed on the -Z direction side of the second top insulator 24, a circuit board 26 is disposed on the -Z direction side of the fluid distribution tube P1, a pair of second contact members (counter contact members) 25 are disposed on the -Z direction side of the circuit board 26, the second bottom insulator 23 is disposed on the -Z direction side of the pair of second contact members 25, and a waterproof member 27 is disposed on the -Z direction side of the second bottom insulator 23.

As shown in FIG. 12, the second bottom insulator 23 includes a flat plate portion 23A extending along an XY plane; at a center portion of the flat plate portion 23A, a cylindrical shaped portion 23B is formed to project and extend from the +Z directional surface of the flat plate portion 23A in the +Z direction, and, in the interior of the cylindrical shaped portion 23B, a passage connection port accommodation portion 23C of cylindrical shape is integrally formed to extend in the Z direction and penetrate the second bottom insulator 23.

The second bottom insulator 23 also includes a protrusion portion 23D projecting from the flat plate portion 23A in the -Z direction, and a pair of recessed contact accommodation portions 23E are formed inside the protrusion portion 23D, the pair of contact accommodation portions 23E opening in the +Z direction and being situated on opposite sides of the cylindrical shaped portion 23B in the Y direction across the cylindrical shaped portion 23B. In a bottom portion of each of the contact accommodation portions 23E, a through-hole 23F is formed.

The flat plate portion 23A is provided, on the -Y direction side of the cylindrical shaped portion 23B, with a pair of positioning pins 23G disposed to be separated from each other in the X direction and projecting in the +Z direction.

At a peripheral portion of the flat plate portion 23A, a peripheral wall portion 23H is formed to project in the +Z direction, and at an outer peripheral portion of the protrusion portion 23D, a circumferential groove 23J is disposed to surround the protrusion portion 23D along an XY plane.

In addition, a cutout 23K in which part of the fluid distribution tube P1 is inserted is formed at the +Y directional end portion of the circumferential wall portion 23H of the second bottom insulator 23.

As shown in FIG. 13, the second top insulator 24 includes a top plate portion 24A extending along an XY plane, and, at a peripheral portion of the top plate portion 24A, a circumferential wall portion 24B is formed to project in the -Z direction. With the top plate portion 24A and the circumferential wall portion 24B, the second top insulator 24 has a box-like shape opening in the -Z direction.

In addition, a cutout 24C through which the fluid distribution tube P1 passes is formed at the +Y directional end portion of the circumferential wall portion 24B of the second top insulator 24.

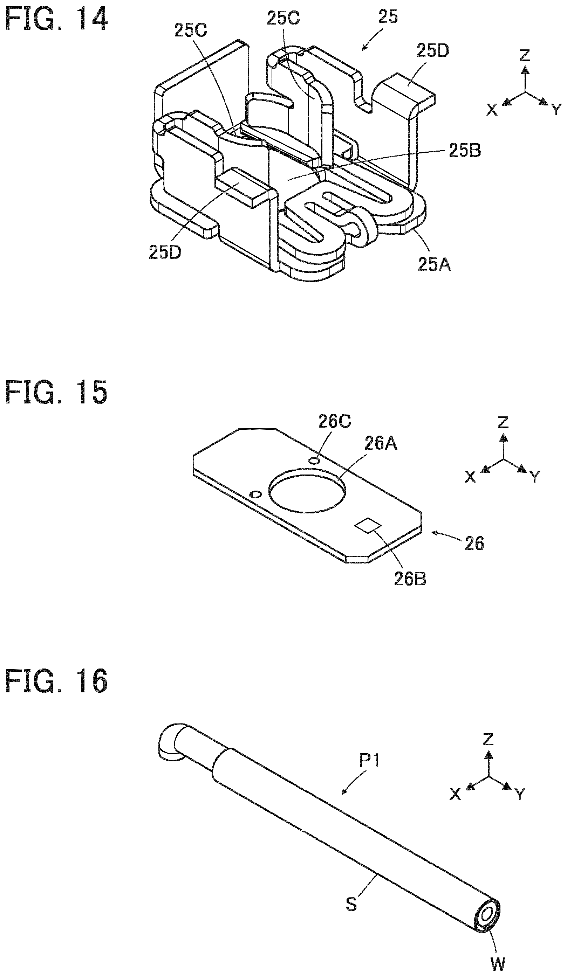

As shown in FIG. 14, the second contact member 25 is made of, for example, a conductive metal sheet, and includes a base portion 25A extending along an XY plane. The base portion 25A is provided with a through-hole 25B through which the tubular portion 15A of the corresponding first contact member 15 of the first contact 11 passes when the first connector 11 and the second connector 21 are fitted with each other.

A pair of contact portions 25C are joined to the base portion 25A, the contact portions 25C making elastic contact with the tubular portion 15A of the first contact member 15. Moreover, the second contact member 25 is provided with fixing portions 25D facing in the +Z direction.

As shown in FIG. 15, the circuit board 26 has a flat plate shape, and at a center portion of the circuit board 26, a through-hole 26A through which the tip of the fluid distribution tube P1 passes is formed.

A connection pad 26B is disposed on the +Y direction side of the through-hole 26A on the +Z directional surface of the circuit board 26, and also on the -Z directional surface of the circuit board 26, disposed is a connection pad (not shown) electrically connected to the connection pad 26B.

The circuit board 26 is further provided, on the -Y direction side of the through-hole 26A, with a pair of positioning holes 26C disposed to be separated from each other in the X direction and penetrating the circuit board 26 in the Z direction.

As shown in FIG. 16, the fluid distribution tube P1 is covered by a casing S; in the casing S, the fluid distribution tube P1 for distributing air, water or another fluid and an electric wire W for transmitting an electric signal are accommodated, thereby configuring a so-called composite cable.

By removing part of the casing S over a predetermined length in the vicinity of the -Y directional end portion of the fluid distribution tube P1, the fluid distribution tube P1 is exposed from the casing S, and the electric wire W is also exposed while not shown in the drawings.

The -Y directional tip of the fluid distribution tube P1 exposed from the casing S is bent toward the -Z direction.

When the second connector 21 is assembled, first, the pair of second contact members 25 are attached to the circuit board 26. In this process, the fixing portions 25D of each second contact member 25 are fixed to connection pads (not shown) disposed on the -Z directional surface of the circuit board 26 by soldering or another means.

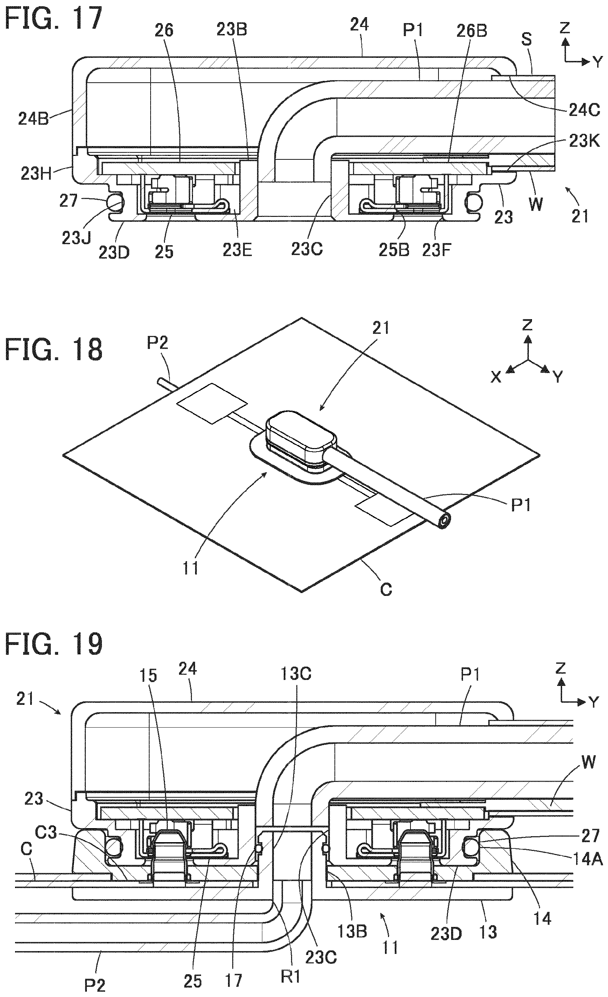

Subsequently, with the pair of positioning pins 23G of the second bottom insulator 23 being passed through the pair of positioning holes 26C of the circuit board 26, the circuit board 26 is disposed inside the circumferential wall portion 23H of the second bottom insulator 23. With this constitution, as shown in FIG. 17, the pair of second contact members 25 fixed to the circuit board 26 are accommodated in the pair of recessed contact accommodation portions 23E of the second bottom insulator 23, and the through-hole 25B of each second contact member 25 is situated at a position overlapping the through-hole 23F of the corresponding contact accommodation portion 23E on the +Z direction side.

In this state, the fluid distribution tube P1 is disposed on the +Z direction side of the second bottom insulator 23, and the -Y directional tip of the fluid distribution tube P1 being bent from the Y direction toward the -Z direction is inserted into the passage connection port accommodation portion 23C of the second bottom insulator 23 and fixed therein. By use of an adhesive, for example, the tip of the fluid distribution tube P1 can be bonded to an inner surface of the cylindrical shaped portion 23B.

In addition, the electric wire W exposed from the casing S in the vicinity of the - Y directional end portion of the fluid distribution tube P1 is connected to the connection pad 26B of the circuit board 26 by soldering or another means, whereby the pair of second contact members 25 are electrically connected to the electric wire W.

Thereafter, the second top insulator 24 is disposed on the +Z direction side of the second bottom insulator 23, and the circumferential wall portion 23H of the second bottom insulator 23 and the circumferential wall portion 24B of the second top insulator 24 are bonded together in abutment with each other in the Z direction, whereby the second top insulator 24 is fixed to the second bottom insulator 23. Moreover, the waterproof member 27 is fitted in the circumferential groove 23J of the protrusion portion 23D of the second bottom insulator 23. The assembling operation of the second connector 21 is thus completed.

It should be noted that the fluid distribution tube P1 extends from the second connector 21 in the +Y direction through the cutout 23K of the second bottom insulator 23 and the cutout 24C of the second top insulator 24.

As shown in FIG. 18, by pressing the second connector 21 from the +Z direction toward the first connector 11 mounted on the mounting object C, the second connector 21 is fitted with the first connector 11, and the connector assembly according to Embodiment 1 is assembled.

At this time, as shown in FIG. 19, the protrusion portion 23D of the second bottom insulator 23 of the second connector 21 is inserted in the second connector accommodation portion 14A of the first top insulator 14 of the first connector 11, and the passage connection port 13B of the first connector 11 is accommodated in the passage connection port accommodation portion 23C of the second connector 21.

As a result, the fluid distribution tube P1 having its tip inserted in the passage connection port accommodation portion 23C communicates with the first distribution port R1 of the first connector 11 via the linking passage 13C of the passage connection port 13B; since the mounting object-side distribution tube P2 is joined to the first distribution port R1, the fluid distribution tube P1 and the mounting object-side distribution tube P2 communicate with each other. Accordingly, with use of a pump or another device (not shown), a fluid such as air or water can be supplied to the mounting object-side distribution tube P2 via the fluid distribution tube P1, or a fluid can be sucked into the fluid distribution tube P1 via the mounting object-side distribution tube P2.

In addition, by pressing the second connector 21 toward the first connector 11, the pair of first contact members 15 of the first connector 11 make contact with the pair of second contact members 25 of the second connector 21 to establish electrical conduction therebetween. Since the first contact member 15 is connected to the wiring portion C3 of the mounting object C and the second contact member 25 is connected to the electric wire W, when the first contact member 15 and the second contact member 25 establish electrical conduction therebetween, the wiring portion C3 of the mounting object C is electrically connected to the electric wire W. In addition, since the electrode C4 is connected to the wiring portion C3 in the mounting object C, the electrode C4 is electrically connected to the electric wire W via the wiring portion C3, the first contact member 15, and the second contact member 25.

Therefore, when fabric of smart clothes is adopted as the mounting object C, biological information of a user obtained by the electrode C4 can be transmitted to, for example, a measurement device via the electric wire W.

As described above, according to Embodiment 1, by merely fitting the second connector 21 with the first connector 11, electrical connection and connection of a fluid passage can be established at a time, thus realizing a connector assembly with high utility.

In addition, since the passage connection port 13B for connection of a fluid passage is integrally formed in the first bottom insulator 13 made of an insulating resin while the passage connection port accommodation portion 23C is integrally formed in the second bottom insulator 23 made of an insulating resin, the manufacturing process of the connector assembly is simplified, whereby the connector assembly can be easily manufactured.

It should be noted that fabric of a garment such as smart clothes can be adopted as the mounting object C, and the connector assembly can be also configured to supply a fluid such as air or water to a user's body surface or suck out sweat, urine, or the like from the user's side by having the fluid distribution tube P1 and the mounting object-side distribution tube P2 communicating with each other.

Since the O-ring 17 is disposed at the outer peripheral portion of the passage connection port 13B, a space between an inner peripheral surface of the passage connection port accommodation portion 23C and an outer peripheral surface of the passage connection port 13B is sealed, and since the waterproof member 27 is disposed at the outer peripheral portion of the protrusion portion 23D of the second bottom insulator 23, a space between an inner peripheral surface of the second connector accommodation portion 14A of the first top insulator 14 and an outer peripheral surface of the protrusion portion 23D of the second bottom insulator 23 is sealed.

### [Modification 1 of Embodiment 1]

In the first connector 11 according to Embodiment 1 described above, the passage connection port 13B is integrally formed in the first bottom insulator 13, but the present invention is not limited to the case.

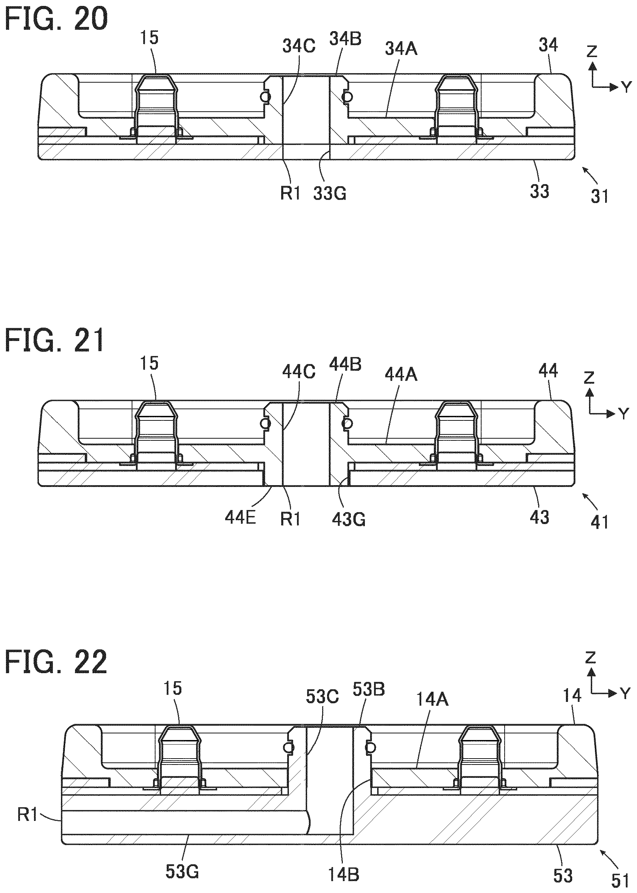

FIG. 20 shows a first connector 31 in Modification 1 of Embodiment 1. The first connector 31 includes a first bottom insulator 33 and a first top insulator 34 each made of an insulating resin, and a passage connection port 34B is integrally formed in the first top insulator 34.

The first top insulator 34 includes a recessed second connector accommodation portion 34A opening in the +Z direction; at a center portion of the second connector accommodation portion 34A, the passage connection port 34B is formed to project in the +Z direction, and in the interior of the passage connection port 34B, a linking passage 34C is formed to penetrate the first top insulator 34 in the Z direction.

In the first bottom insulator 33, an opening portion 33G is formed to communicate with the linking passage 34C on the -Z direction side thereof, the opening portion 33G and the linking passage 34C having the same inside diameter, and an opening end portion on the -Z direction side of the opening portion 33G constitutes the first distribution port R1 opening toward an outside of the first connector 31.

The first bottom insulator 33 and the first top insulator 34 respectively have the same configurations as those of the first bottom insulator 13 and the first top insulator 14 of the first connector 11 in Embodiment 1 except that, in place of the passage connection port 13B being formed in the first bottom insulator 13, the passage connection port 34B is formed in the first top insulator 34, and the opening portion 33G is formed in the first bottom insulator 33.

Even when the first connector 31 configured as above is used in place of the first connector 11, by merely fitting the second connector 21 with the first connector 31, electrical connection and connection of a fluid passage can be established at a time as with Embodiment 1.

### [Modification 2 of Embodiment 1]

FIG. 21 shows a first connector 41 in Modification 2 of Embodiment 1. The first connector 41 includes a first bottom insulator 43 and a first top insulator 44 each made of an insulating resin, and a passage connection port 44B is integrally formed in the first top insulator 44.

The first top insulator 44 includes a recessed second connector accommodation portion 44A opening in the +Z direction, and, at a center portion of the second connector accommodation portion 44A, a passage connection port 44B is formed to project in the +Z direction.

In addition, on the -Z direction side of the passage connection port 44B, a cylindrical shaped portion 44E is formed to project in the -Z direction from the first top insulator 44, and in the interiors of the passage connection port 44B and the cylindrical shaped portion 44E, a linking passage 44C is formed to penetrate the first top insulator 44 in the Z direction.

The cylindrical shaped portion 44E of the first top insulator 44 is inserted in an opening portion 43G formed in the first bottom insulator 43, and an end surface of the cylindrical shaped portion 44E facing in the -Z direction is exposed on the -Z direction side of the first bottom insulator 43. In other words, the linking passage 44C penetrates the first connector 41 in the Z direction, and an opening end portion on the -Z direction side of the cylindrical shaped portion 44E constitutes the first distribution port R1 opening toward an outside of the first connector 41.

The first bottom insulator 43 and the first top insulator 44 respectively have the same configurations as those of the first bottom insulator 13 and the first top insulator 14 of the first connector 11 in Embodiment 1 except that, in place of the passage connection port 13B being formed in the first bottom insulator 13, the passage connection port 44B and the cylindrical shaped portion 44E are formed in the first top insulator 44, and the opening portion 43G is formed in the first bottom insulator 43.

Even when the first connector 41 configured as above is used in place of the first connector 11, by merely fitting the second connector 21 with the first connector 41, electrical connection and connection of a fluid passage can be established at a time as with Embodiment 1.

### [Modification 3 of Embodiment 1]

FIG. 22 shows a first connector 51 in Modification 3 of Embodiment 1. The first connector 51 includes a first bottom insulator 53 and the first top insulator 14 each made of an insulating resin, and a passage connection port 53B is integrally formed in the first bottom insulator 53. The first top insulator 14 is the same as the first top insulator 14 in the first connector 11 of Embodiment 1.

The passage connection port 53B penetrates the connection port through-hole 14B of the first top insulator 14 to project in the +Z direction from the second connector accommodation portion 14A of the first top insulator 14.

While in the interior of the passage connection port 53B, a linking passage 53C is formed to extend in the -Z direction from the +Z directional end portion of the passage connection port 53B, the linking passage 53C does not penetrate the first bottom insulator 53 in the Z direction but communicates with a first passage 53G extending inside the first bottom insulator 53 in the Y direction.

The first passage 53G is opened at the -Y directional end portion of the first bottom insulator 53, and an opening end portion of the first passage 53G constitutes the first distribution port R1 opening toward an outside of the first connector 51.

Although not shown in the drawings, the mounting object-side distribution tube P2 as used in Embodiment 1 can be joined to the first distribution port R1.

The first bottom insulator 53 has the same configuration as that of the first bottom insulator 13 in the first connector 11 of Embodiment 1 except that, in place of the passage connection port 13B and the linking passage 13C, the passage connection port 53B, the linking passage 53C, and the first passage 53G are formed.

Even when the first connector 51 configured as above is used in place of the first connector 11, by merely fitting the second connector 21 with the first connector 51, electrical connection and connection of a fluid passage can be established at a time as with Embodiment 1.

In addition, it is possible that in the first bottom insulator 53, a plurality of first passages 53G are formed to each communicate with the linking passage 53C, and a plurality of first distribution ports R1 are formed to correspond to the plurality of first passages 53G. With this constitution, for instance, a fluid such as air or water can be supplied through the fluid distribution tube P1 and distributed from the first distribution ports R1.

Moreover, the linking passage 53C may be formed to penetrate the first bottom insulator 53 in the Z direction while the first passage 53G extending inside the first bottom insulator 53 in the Y direction communicates with the linking passage 53C.

### [Embodiment 2]

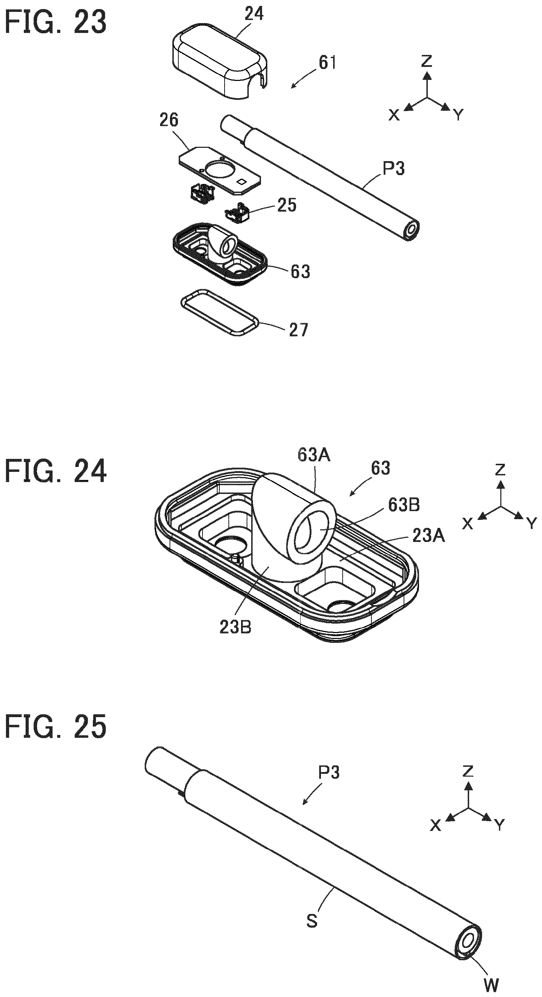

FIG. 23 shows an assembly view of a second connector 61 used in a connector assembly according to Embodiment 2.

A fluid distribution tube P3 is disposed on the -Z direction side of the second top insulator 24, the circuit board 26 is disposed on the -Z direction side of the fluid distribution tube P3, the pair of second contact members 25 are disposed on the -Z direction side of the circuit board 26, a second bottom insulator 63 is disposed on the -Z direction side of the pair of second contact members 25, and the waterproof member 27 is disposed on the -Z direction side of the second bottom insulator 63.

The second top insulator 24, the circuit board 26, the second contact member 25, and the waterproof member 27 are the same members with the same reference numbers as those in the second connector 21 of Embodiment 1.

As shown in FIG. 24, the second bottom insulator 63 is made of an insulating resin and includes a bent tube portion 63A integrally formed on the +Z direction side of the cylindrical shaped portion 23B that is formed to project at a center portion of the flat plate portion 23A. In the interior of the bent tube portion 63A, formed is a bent passage 63B that is bent such that one end thereof faces in the +Y direction orthogonal to the Z direction being the fitting direction, and the other end thereof faces in the -Z direction.

The second bottom insulator 63 has the same configuration as that of the second bottom insulator 23 in the second connector 21 of Embodiment 1 except that the bent tube portion 63A is provided.

As shown in FIG. 25, the fluid distribution tube P3 is covered by the casing S, and by removing part of the casing S over a predetermined length in the vicinity of the - Y directional end portion of the fluid distribution tube P3, the fluid distribution tube P3 is exposed from the casing S, and the -Y directional tip of the fluid distribution tube P3 is not bent but linearly extends in the -Y direction.

The fluid distribution tube P3 has the same configuration as that of the fluid distribution tube P1 used in Embodiment 1 except that the -Y directional tip thereof is not bent but linearly extends in the -Y direction.

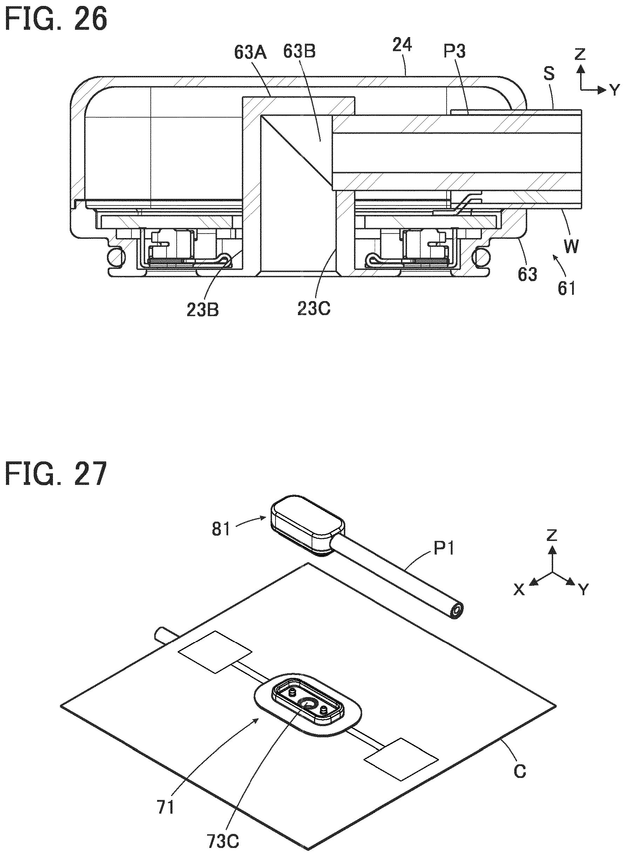

When the second connector 61 is assembled, as with the assembly of the second connector 21 of Embodiment 1, the circuit board 26 to which the pair of second contact members 25 are attached is disposed inside the second bottom insulator 63, and thereafter the -Y directional end portion of the fluid distribution tube P3 is fixed to an end portion, facing in the +Y direction, of the bent tube portion 63A of the second bottom insulator 63 as shown in FIG. 26.

As a result, the fluid distribution tube P3 communicates with the passage connection port accommodation portion 23C formed in the interior of the cylindrical shaped portion 23B via the bent passage 63B of the bent tube portion 63A.

The -Y directional end portion of the fluid distribution tube P3 is inserted in the inside of the +Y directional end portion of the bent tube portion 63A and bonded to an inner surface of the bent tube portion 63A by use of, for example, an adhesive.

In addition, the electric wire W exposed from the casing S of the fluid distribution tube P3 is connected to the connection pad 26B of the circuit board 26, and, furthermore, the second top insulator 24 is fixed on the +Z direction side of the second bottom insulator 63, whereby the assembling operation of the second connector 61 is completed.

By fitting the second connector 61 as above with the first connector 11 of Embodiment 1, the connector assembly according to Embodiment 2 is assembled. As with Embodiment 1, by merely fitting the second connector 61 with the first connector 11, electrical connection and connection of a fluid passage can be established at a time, and since the passage connection port accommodation portion 23C is integrally formed in the second bottom insulator 63 made of an insulating resin, the connector assembly can be easily manufactured also in Embodiment 2.

It should be noted that the second connector 61 in Embodiment 2 can be also fitted with any one of the first connectors 31 to 51 shown in FIGS. 20 to 22, whereby the connector assembly can be assembled.

### [Embodiment 3]

In Embodiment 1, the first connector 11 to be mounted on the mounting object C includes the passage connection port 13B, and the second connector 21 to be attached to the tip of the fluid distribution tube P1 includes the passage connection port accommodation portion 23C, but the present invention is not limited to the case.

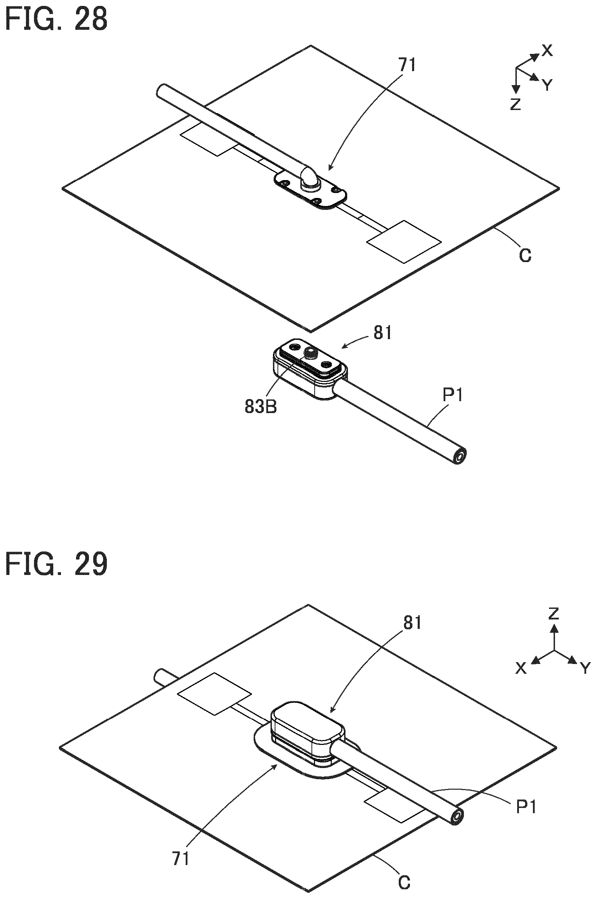

FIGS. 27 and 28 show a connector assembly according to Embodiment 3 in the non-fitted state. The connector assembly includes a first connector 71 to be mounted on the mounting object C and a second connector 81 to be attached to the -Y directional tip of the fluid distribution tube P1, and the first connector 71 includes a passage connection port accommodation portion 73C, and the second connector 81 includes a passage connection port 83B.

As shown in FIG. 29, by pressing the second connector 81 from the +Z direction toward the first connector 71, the second connector 81 is fitted with the first connector 71, and the connector assembly according to Embodiment 3 is assembled.

As shown in FIG. 30, the first connector 71 includes a first bottom insulator 73 and a first top insulator 74 each made of an insulating resin. The first bottom insulator 73 includes a flat plate portion 73A extending along an XY plane, and at a center portion of the flat plate portion 73A, the passage connection port accommodation portion 73C of recess shape is integrally formed to extend in the Z direction and penetrate the first bottom insulator 73. On the -Z direction side of the passage connection port accommodation portion 73C, a cylindrical shaped portion 73B is formed to project from the first bottom insulator 73 in the -Z direction, and an opening end portion on the -Z direction side of the cylindrical shaped portion 73B constitutes the first distribution port R1 opening toward an outside of the first connector 71.

The first bottom insulator 73 has the same configuration as that of the first bottom insulator 13 in the first connector 11 of Embodiment 1 except that, in place of the passage connection port 13B, the passage connection port accommodation portion 73C is provided, while the first top insulator 74 has the same configuration as that of the first top insulator 14 in the first connector 11 of Embodiment 1.

In addition, the pair of first contact members 15 are attached to the first bottom insulator 73.

The second connector 81 includes a second bottom insulator 83 and a second top insulator 84 each made of an insulating resin. At a center portion of the second bottom insulator 83, the passage connection port 83B of cylindrical shape is integrally formed to extend in the -Z direction, and the passage connection port 83B is provided in its interior with a linking passage 83C extending in the Z direction and penetrating the second bottom insulator 83.

The second bottom insulator 83 has the same configuration as that of the second bottom insulator 23 in the second connector 21 of Embodiment 1 except that, in place of the passage connection port accommodation portion 23C, the passage connection port 83B is provided, while the second top insulator 84 has the same configuration as that of the second top insulator 24 in the second connector 21 of Embodiment 1.

In addition, the pair of second contact members 25 are attached to the second bottom insulator 83.

The tip of the fluid distribution tube P1 bent toward the -Z direction is fixed to the +Z directional end portion of the passage connection port 83B of the second bottom insulator 83 of the second connector 81. The tip of the fluid distribution tube P1 is bonded to the passage connection port 83B by use of an adhesive, for example.

Moreover, an end portion of the mounting object-side distribution tube P4 is inserted from the -Z direction in the passage connection port accommodation portion 73C of the first bottom insulator 73 of the first connector 71, whereby the mounting object-side distribution tube P4 is joined to the first distribution port R1 opening toward the outside of the first connector 71.

The second connector 81 is pressed from the +Z direction toward the first connector 71, the passage connection port 83B of the second connector 81 is thus accommodated in the passage connection port accommodation portion 73C of the first connector 71, and, as a result, the fluid distribution tube P1 communicates with the mounting object-side distribution tube P4 via the linking passage 83C of the passage connection port 83B. Accordingly, with use of a pump or another device (not shown), a fluid such as air or water can be supplied to the mounting object-side distribution tube P4 via the fluid distribution tube P1, or a fluid can be sucked into the fluid distribution tube P1 via the mounting object-side distribution tube P4.

In addition, by fitting the second connector 81 with the first connector 71, the pair of first contact members 15 of the first connector 71 make contact with the pair of second contact members 25 of the second connector 81 to establish electrical conduction therebetween.

Therefore, when fabric of smart clothes is adopted as the mounting object C, obtained biological information of a user can be transmitted to, for example, a measurement device via the electric wire W.

As described above, even when the first connector 71 includes the passage connection port accommodation portion 73C while the second connector 81 includes the passage connection port 83B, by merely fitting the second connector 81 with the first connector 71, electrical connection and connection of a fluid passage can be established at a time as with Embodiment 1.

In addition, since the passage connection port accommodation portion 73C is integrally formed in the first bottom insulator 73 made of an insulating resin while the passage connection port 83B is integrally formed in the second bottom insulator 83 made of an insulating resin, the manufacturing process of the connector assembly is simplified, whereby the connector assembly can be easily manufactured.

It should be noted that, in also Embodiment 3, the connector assembly can be configured such that the second connector 81 includes the bent tube portion 63A used in Embodiment 2, and the fluid distribution tube P3 is fixed to an end portion of the bent tube portion 63A.

In Embodiments 1 to 3 described above, the mounting object C includes the pair of wiring portions C3 and the pair of electrodes C4 formed of embroidery using embroidery threads made of a conductive thread; however, the connector assembly can be also configured such that a wiring portion and an electrode each having a conductive layer formed on a surface of an insulating resin film by printing or another method are attached to a surface of the mounting object C.

Furthermore, a pressure sensor or other various sensors may be connected to the wiring portion C3 in place of the electrode C4.

In Embodiments 1 to 3 described above, the pair of first contact members 15 and the pair of second contact members 25 are used; however, it suffices if at least one first contact member 15 and at least one second contact member 25 are provided, and the number of the first contact members 15 or of the second contact members 25 is not limited.

## Claims

1. A connector (11, 31, 41, 51, 71) which is configured to be mounted on a mounting object (C) and to which a counter connector (21, 61, 81) is to be fitted along a fitting direction, the mounting object being flexible and being provided with a wiring portion (C3), the connector comprising:
a housing (12) that is configured to be attached to the mounting object, includes a distribution port (R1) opening toward an outside of the connector, and is made of an insulating resin, wherein the housing is composed of a top insulator (14, 34, 44, 74) and a bottom insulator (13, 33, 43, 53, 73), the top insulator being provided with a connector accommodation portion (14A, 34A, 44A) for accommodating part of the counter connector when the connector is fitted to the counter connector, and the bottom insulator being joined to the top insulator, and
a contact member (15) that is conductive and is held by the housing and is configured to be electrically connected to the wiring portion,
wherein one of following options (i) and (ii) applies:
(i) a passage connection port (13B, 34B, 44B, 53B, 83B) of tubular shape is integrally formed in the housing, the passage connection port extending along the fitting direction and including in its interior a linking passage (13C, 34C, 44C, 53C, 83C) extending along the fitting direction,
(ii) a passage connection port accommodation portion (73C) of recess shape is integrally formed in the housing, the passage connection port accommodation portion extending along the fitting direction and being configured to accommodate a passage connection port of the counter connector, and
wherein the connector is configured such that, when the counter connector is fitted with the connector, the following configurations apply:
the contact member (15) is electrically connected to a counter contact member (25) of the counter connector, and, in an implementation of the above option (i), the passage connection port is accommodated in a passage connection port accommodation portion (23C, 73C) of the counter connector or, in an implementation of the above option (ii), a passage connection port of the counter connector is accommodated in the passage connection port accommodation portion (73C), whereby the distribution port (R1) communicates, via the linking passage (13C, 34C, 44C, 53C, 83C), with a fluid distribution tube (P1, P3) attached to the counter connector (21, 61, 81), and
the mounting object is held between the top insulator and the bottom insulator, whereby the connector (11, 31, 41, 51, 71) is mounted on the mounting object.

2. A connector assembly comprising a connector according to claim 1 forming a first connector and further comprising a counter connector forming a second connector, wherein the second connector (21, 61, 81) is fitted to the first connector (11, 31, 41, 51, 71) along a fitting direction, the second connector being attached to a tip of a fluid distribution tube (P1, P3), and the first connector being mounted on a mounting object (C) that is flexible and is provided with a wiring portion (C3),
wherein the housing of the first connector forms a first housing (12), includes the distribution port forming a first distribution port (R1) and is composed of the top insulator forming a first top insulator (14, 34, 44, 74) and the bottom insulator forming a first bottom insulator (13, 33, 43, 53, 73); and
wherein the contact member of the first connector forms a first contact member (15),
wherein the second connector includes:
a second housing (22) that is configured to be attached to the tip of the fluid distribution tube, opposes the first housing, and is made of an insulating resin; and
a second contact member (25) that is conductive, is held by the second housing, and corresponds to the first contact member,
wherein, when the passage connection port (13B, 34B, 44B, 53B, 83B) is integrally formed in the first housing, a passage connection port accommodation portion (23C, 73C) of recess shape is integrally formed in the second housing, the passage connection port accommodation portion extending along the fitting direction and being configured to accommodate the passage connection port, and
wherein alternatively, when the passage connection port accommodation portion (23C, 73C) is integrally formed in the first housing, a passage connection port (13B, 34B, 44B, 53B, 83B) of tubular shape is integrally formed in the second housing, the passage connection port extending along the fitting direction and including in its interior a linking passage (13C, 34C, 44C, 53C, 83C) extending along the fitting direction, and
when the second connector is fitted with the first connector, the first contact member (15) is electrically connected to the second contact member (25), and the passage connection port is accommodated in the passage connection port accommodation portion whereby the first distribution port (R1) communicates with the fluid distribution tube (P1, P3) via the linking passage (13C, 34C, 44C, 53C, 83C),
wherein the connector accommodation portion of the first top insulator forms a second connector accommodation portion (14A, 34A, 44A) for accommodating part of the second connector when the first connector is fitted to the second connector, and the first bottom insulator being joined to the first top insulator, and
the mounting object is held between the first top insulator and the first bottom insulator, whereby the first connector (11, 31, 41, 51, 71) is mounted on the mounting object.

3. The connector assembly according to claim 2,
wherein the passage connection port (13B, 34B, 44B, 53B) is formed in the first housing (12), and
the passage connection port accommodation portion (23C) is formed in the second housing (22).

4. The connector assembly according to claim 3, wherein the passage connection port (13B, 53B) is formed in the first bottom insulator (13, 53).

5. The connector assembly according to claim 3, wherein the passage connection port (34B, 44B) is formed in the first top insulator (34, 44).

6. The connector assembly according to any one of claims 3-5,
wherein the linking passage (13C, 34C, 44C) penetrates the first housing (12) in the fitting direction, and
the first distribution port (R1) is disposed at an end portion of the linking passage.

7. The connector assembly according to claim 3 or 4, wherein the first housing (12) includes a first passage (53G) extending in a direction orthogonal to the fitting direction and communicating the linking passage and the first distribution port with each other.

8. The connector assembly according to any one of claims 3-7, wherein the tip of the fluid distribution tube (P1, P3) is fixed in the passage connection port accommodation portion (23C) of the second housing (22).

9. The connector assembly according to claim 8, wherein the tip of the fluid distribution tube (P1) is fixed in the passage connection port accommodation portion (23C) while being bent toward the fitting direction from a direction orthogonal to the fitting direction.

10. The connector assembly according to claim 3,
wherein the tip of the fluid distribution tube (P3) is fixed in the second housing (22) while extending in a direction orthogonal to the fitting direction,
the second housing (22) includes a bent passage (63B) with one end thereof facing in the direction orthogonal to the fitting direction and another end thereof facing in the fitting direction, and
the tip of the fluid distribution tube (P3) and the passage connection port accommodation portion (23C) communicate each other via the bent passage (63B).

11. The connector assembly according to claim 2,
wherein the passage connection port (83B) is formed in the second housing (22), and
the passage connection port accommodation portion (73C) is formed in the first housing (12).

12. The connector assembly according to any one of claims 2-11,
wherein the first contact member (15) has a tubular portion (15A) extending along the fitting direction and provided in its interior with a recessed portion (15C),
the first bottom insulator (13, 33, 43, 53, 73) includes a projection (13E) to be inserted in the recessed portion of the first contact member, and
the projection is press-fitted into the recessed portion with the wiring portion of the mounting object being held therebetween, whereby the first contact member (15) is electrically connected to the wiring portion (C3).

13. The connector assembly according to any one of claims 2-12, wherein the first connector (11, 31, 41, 51, 71) includes a reinforcement sheet (16) held by the first housing and extending along a surface of the mounting object to reach an outside of an outer peripheral portion of the first housing.

14. The connector assembly according to any one of claims 2-13,
wherein a mounting object-side distribution tube (P2, P4) is joined to the first distribution port of the first housing, and
the mounting object-side distribution tube communicates with the fluid distribution tube (P1, P3) via the linking passage.

## Patentansprüche

1. Verbinder (11, 31, 41, 51, 71), der konfiguriert ist, um an einem Montageobjekt (C) montiert zu werden, und an dem ein Gegenverbinder (21, 61, 81) entlang einer Montagerichtung zu montieren ist, wobei das Montageobjekt flexibel ist und mit einem Verdrahtungsabschnitt (C3) versehen ist, wobei der Verbinder umfasst:
ein Gehäuse (12), das konfiguriert ist, um an dem Montageobjekt befestigt zu werden, einen Verteilungsanschluss (R1) enthält, der sich zu einer Außenseite des Verbinders öffnet, und aus einem isolierenden Harz hergestellt ist, wobei das Gehäuse aus einem oberen Isolator (14, 34, 44, 74) und einem unteren Isolator (13, 33, 43, 53, 73) zusammengesetzt ist, wobei der obere Isolator mit einem Verbinderaufnahmeabschnitt (14A, 34A, 44A) zum Aufnehmen eines Teils des Gegenverbinders versehen ist, wenn der Verbinder an dem Gegenverbinder montiert ist, und der untere Isolator mit dem oberen Isolator verbunden ist, und
ein Kontaktelement (15), das leitfähig ist und durch das Gehäuse gehalten wird und konfiguriert ist, um elektrisch mit dem Verdrahtungsabschnitt verbunden zu werden,
wobei eine folgender Optionen (i) und (ii) gilt:
(i) ein Durchgangsverbindungsanschluss (13B, 34B, 44B, 53B, 83B) von Röhrenform ist integral in dem Gehäuse ausgebildet, wobei sich der Durchgangsverbindungsanschluss entlang der Montagerichtung erstreckt und in seinem Inneren einen Verbindungsdurchgang (13C, 34C, 44C, 53C, 83C) enthält, der sich entlang der Montagerichtung erstreckt,
(ii) ein Durchgangsverbindungsanschlussaufnahmeabschnitt (73C) von Aussparungsform ist integral in dem Gehäuse ausgebildet, wobei sich der Durchgangsverbindungsanschlussaufnahmeabschnitt entlang der Montagerichtung erstreckt und konfiguriert ist, um einen Durchgangsverbindungsanschluss des Gegenverbinders aufzunehmen, und
wobei der Verbinder so konfiguriert ist, dass, wenn der Gegenverbinder mit dem Verbinder montiert ist, die folgenden Konfigurationen gelten:
das Kontaktelement (15) ist elektrisch mit einem Gegenkontaktelement (25) des Gegenverbinders verbunden, und in einer Implementierung der obigen Option (i) ist der Durchgangsverbindungsanschluss in einem Durchgangsverbindungsanschlussaufnahmeabschnitt (23C, 73C) des Gegenverbinders aufgenommen oder in einer Implementierung der obigen Option (ii) ist ein Durchgangsverbindungsanschluss des Gegenverbinders in dem Durchgangsverbindungsanschlussaufnahmeabschnitt (73C) aufgenommen, wodurch der Verteilungsanschluss (R1) über den Verbindungsdurchgang (13C, 34C, 44C, 53C, 83C) mit einem Fluidverteilungsrohr (P1, P3) kommuniziert, das an dem Gegenverbinder (21, 61, 81) angebracht ist, und
das Montageobjekt wird zwischen dem oberen Isolator und dem unteren Isolator gehalten, wodurch der Verbinder (11, 31, 41, 51, 71) an dem Montageobjekt montiert ist.

2. Verbinderanordnung, umfassend einen Verbinder nach Anspruch 1, der einen ersten Verbinder bildet, und ferner umfassend einen Gegenverbinder, der einen zweiten Verbinder bildet, wobei der zweite Verbinder (21, 61, 81) an dem ersten Verbinder (11, 31, 41, 51, 71) entlang einer Montagerichtung montiert ist, wobei der zweite Verbinder an einer Spitze eines Fluidverteilungsrohrs (P1, P3) angebracht ist, und der erste Verbinder an einem Montageobjekt (C) montiert ist, das flexibel ist und mit einem Verdrahtungsabschnitt (C3) versehen ist,
wobei das Gehäuse des ersten Verbinders ein erstes Gehäuse (12) bildet, den Verteilungsanschluss enthält, der einen ersten Verteilungsanschluss (R1) bildet, und aus dem oberen Isolator, der einen ersten oberen Isolator (14, 34, 44, 74) bildet, und dem unteren Isolator, der einen ersten unteren Isolator (13, 33, 43, 53, 73) bildet, zusammengesetzt ist; und
wobei das Kontaktelement des ersten Verbinders ein erstes Kontaktelement (15) bildet,
wobei der zweite Verbinder enthält:
ein zweites Gehäuse (22), das konfiguriert ist, um an der Spitze des Fluidverteilungsrohrs angebracht zu werden, dem ersten Gehäuse gegenüberliegt und aus einem isolierenden Harz hergestellt ist; und
ein zweites Kontaktelement (25), das leitfähig ist, durch das zweite Gehäuse gehalten wird und dem ersten Kontaktelement entspricht,
wobei, wenn der Durchgangsverbindungsanschluss (13B, 34B, 44B, 53B, 83B) integral in dem ersten Gehäuse ausgebildet ist, ein Durchgangsverbindungsanschlussaufnahmeabschnitt (23C, 73C) von Aussparungsform integral in dem zweiten Gehäuse ausgebildet ist, wobei sich der Durchgangsverbindungsanschlussaufnahmeabschnitt entlang der Montagerichtung erstreckt und konfiguriert ist, um den Durchgangsverbindungsanschluss aufzunehmen, und
wobei alternativ, wenn der Durchgangsverbindungsanschlussaufnahmeabschnitt (23C, 73C) integral in dem ersten Gehäuse ausgebildet ist, ein Durchgangsverbindungsanschluss (13B, 34B, 44B, 53B, 83B) von Röhrenform integral in dem zweiten Gehäuse ausgebildet ist, wobei sich der Durchgangsverbindungsanschluss entlang der Montagerichtung erstreckt und in ihrem Inneren einen Verbindungsdurchgang (13C, 34C, 44C, 53C, 83C) enthält, der sich entlang der Montagerichtung erstreckt, und
wenn der zweite Verbinder mit dem ersten Verbinder montiert ist, ist das erste Kontaktelement (15) elektrisch mit dem zweiten Kontaktelement (25) verbunden, und der Durchgangsverbindungsanschluss ist in dem Durchgangsverbindungsanschlussaufnahmeabschnitt aufgenommen, wodurch der erste Verteilungsanschluss (R1) mit dem Fluidverteilungsrohr (P1, P3) über den Verbindungsdurchgang (13C, 34C, 44C, 53C, 83C) kommuniziert,
wobei der Verbinderaufnahmeabschnitt des ersten oberen Isolators einen zweiten Verbinderaufnahmeabschnitt (14A, 34A, 44A) zum Aufnehmen eines Teils des zweiten Verbinders bildet, wenn der erste Verbinder an dem zweiten Verbinder montiert ist, und der erste untere Isolator mit dem ersten oberen Isolator verbunden ist, und
das Montageobjekt zwischen dem ersten oberen Isolator und dem ersten unteren Isolator gehalten wird, wodurch der erste Verbinder (11, 31, 41, 51, 71) an dem Montageobjekt montiert ist.

3. Verbinderanordnung nach Anspruch 2,
wobei der Durchgangsverbindungsanschluss (13B, 34B, 44B, 53B) in dem ersten Gehäuse (12) ausgebildet ist, und
der Durchgangsverbindungsanschlussaufnahmeabschnitt (23C) in dem zweiten Gehäuse (22) ausgebildet ist.

4. Verbinderanordnung nach Anspruch 3, wobei der Durchgangsverbindungsanschluss (13B, 53B) in dem ersten unteren Isolator (13, 53) ausgebildet ist.

5. Verbinderanordnung nach Anspruch 3, wobei der Durchgangsverbindungsanschluss (34B, 44B) in dem ersten oberen Isolator (34, 44) ausgebildet ist.

6. Verbinderanordnung nach einem der Ansprüche 3 bis 5,
wobei der Verbindungsdurchgang (13C, 34C, 44C) das erste Gehäuse (12) in der Montagerichtung durchdringt, und
der erste Verteilungsanschluss (R1) an einem Endabschnitt des Verbindungsdurchgangs angeordnet ist.

7. Verbinderanordnung nach Anspruch 3 oder 4, wobei das erste Gehäuse (12) einen ersten Durchgang (53G) enthält, der sich in einer Richtung orthogonal zu der Montagerichtung erstreckt und den Verbindungsdurchgang und den ersten Verteilungsanschluss miteinander verbindet.

8. Verbinderanordnung nach einem der Ansprüche 3-7, wobei die Spitze des Fluidverteilungsrohrs (P1, P3) in dem Durchgangsverbindungsanschlussaufnahmeabschnitt (23C) des zweiten Gehäuses (22) befestigt ist.

9. Verbinderanordnung nach Anspruch 8, wobei die Spitze des Fluidverteilungsrohrs (P1) in dem Durchgangsverbindungsanschlussaufnahmeabschnitt (23C) befestigt ist, während sie zu der Montagerichtung aus einer Richtung orthogonal zu der Montagerichtung gebogen ist.

10. Verbinderanordnung nach Anspruch 3,
wobei die Spitze des Fluidverteilungsrohrs (P3) in dem zweiten Gehäuse (22) befestigt ist, während sie sich in einer Richtung orthogonal zu der Montagerichtung erstreckt,
das zweite Gehäuse (22) einen gebogenen Durchgang (63B) enthält, wobei ein Ende davon in der Richtung orthogonal zu der Montagerichtung zugewandt ist und ein anderes Ende davon in der Montagerichtung zugewandt ist, und
die Spitze des Fluidverteilungsrohrs (P3) und der Durchgangsverbindungsanschlussaufnahmeabschnitt (23C) miteinander über den gebogenen Durchgang (63B) kommunizieren.

11. Verbinderanordnung nach Anspruch 2,
wobei der Durchgangsverbindungsanschluss (83B) in dem zweiten Gehäuse (22) ausgebildet ist, und
der Durchgangsverbindungsanschlussaufnahmeabschnitt (73C) in dem ersten Gehäuse (12) ausgebildet ist.

12. Verbinderanordnung nach einem der Ansprüche 2 bis 11,
wobei das erste Kontaktelement (15) einen Röhrenabschnitt (15A) aufweist, der sich entlang der Montagerichtung erstreckt und in seinem Inneren mit einem vertieften Abschnitt (15C) versehen ist,
der erste untere Isolator (13, 33, 43, 53, 73) einen Vorsprung (13E) enthält, um in den vertieften Abschnitt des ersten Kontaktelements eingesetzt zu werden, und
der Vorsprung in den vertieften Abschnitt pressgepasst wird, wobei der Verdrahtungsabschnitt des Montageobjekts dazwischen gehalten ist, wodurch das erste Kontaktelement (15) elektrisch mit dem Verdrahtungsabschnitt (C3) verbunden ist.

13. Verbinderanordnung nach einem der Ansprüche 2 bis 12, wobei der erste Verbinder (11, 31, 41, 51, 71) eine Verstärkungsplatte (16) enthält, die durch das erste Gehäuse gehalten wird und sich entlang einer Oberfläche des Montageobjekts erstreckt, um eine Außenseite eines Außenumfangsabschnitts des ersten Gehäuses zu erreichen.

14. Verbinderanordnung nach einem der Ansprüche 2 bis 13,
wobei ein montageobjektseitiges Verteilungsrohr (P2, P4) mit dem ersten Verteilungsanschluss des ersten Gehäuses verbunden ist, und
das montageobjektseitige Verteilungsrohr mit dem Fluidverteilungsrohr (P1, P3) über den Verbindungsdurchgang kommuniziert.

## Revendications

1. Connecteur (11, 31, 41, 51, 71) qui est configuré pour être monté sur un objet de montage (C) et sur lequel un contre-connecteur (21, 61, 81) doit être monté le long d'une direction de montage, l'objet de montage étant flexible et étant pourvu d'une partie de câblage (C3), le connecteur comprenant :
un boîtier (12) qui est configuré pour être fixé à l'objet de montage, comprend un orifice de distribution (R1) s'ouvrant vers un extérieur du connecteur, et est constitué d'une résine isolante, dans lequel le boîtier est composé d'un isolant supérieur (14, 34, 44, 74) et d'un isolant inférieur (13, 33, 43, 53, 73), l'isolant supérieur étant pourvu d'une partie de logement de connecteur (14A, 34A, 44A) pour loger une partie du contre-connecteur lorsque le connecteur est monté sur le contre-connecteur, et l'isolant inférieur étant joint à l'isolant supérieur, et
un élément de contact (15) qui est conductible et est maintenu par le boîtier et est configuré pour être électriquement connecté à la partie de câblage,
dans lequel l'une des options (i) et (ii) suivantes s'applique :
(i) un orifice de connexion de passage (13B, 34B, 44B, 53B, 83B) de forme tubulaire est formé d'un seul tenant dans le boîtier, l'orifice de connexion de passage s'étendant le long de la direction de montage et comprenant dans son intérieur un passage de liaison (13C, 34C, 44C, 53C, 83C) s'étendant le long de la direction de montage,
(ii) une partie de logement d'orifice de connexion de passage (73C) de forme évidée est formée d'un seul tenant dans le boîtier, la partie de logement d'orifice de connexion de passage s'étendant le long de la direction de montage et étant configurée pour loger un orifice de connexion de passage du contre-connecteur, et
dans lequel le connecteur est configuré de sorte que, lorsque le contre-connecteur est monté avec le connecteur, les configurations suivantes s'appliquent :
l'élément de contact (15) est électriquement connecté à un contre-élément de contact (25) du contre-connecteur, et, dans une mise en œuvre de l'option (i) ci-dessus, l'orifice de connexion de passage est logé dans une partie de logement d'orifice de connexion de passage (23C, 73C) du contre-connecteur ou, dans une mise en œuvre de l'option (ii) ci-dessus, un orifice de connexion de passage du contre-connecteur est logé dans la partie de logement d'orifice de connexion de passage (73C), moyennant quoi l'orifice de distribution (R1) communique, via le passage de liaison (13C, 34C, 44C, 53C, 83C), avec un tube de distribution de fluide (P1, P3) fixé au contre-connecteur (21, 61, 81), et
l'objet de montage est maintenu entre l'isolant supérieur et l'isolant inférieur, moyennant quoi le connecteur (11, 31, 41, 51, 71) est monté sur l'objet de montage.

2. Ensemble connecteur comprenant un connecteur selon la revendication 1 formant un premier connecteur et comprenant en outre un contre-connecteur formant un second connecteur, dans lequel le second connecteur (21, 61, 81) est monté sur le premier connecteur (11, 31, 41, 51, 71) le long d'une direction de montage, le second connecteur étant fixé à une pointe d'un tube de distribution de fluide (P1, P3), et le premier connecteur étant monté sur un objet de montage (C) qui est flexible et est pourvu d'une partie de câblage (C3),
dans lequel le boîtier du premier connecteur forme un premier boîtier (12), comprend l'orifice de distribution formant un premier orifice de distribution (R1) et est composé de l'isolant supérieur formant un premier isolant supérieur (14, 34, 44, 74) et de l'isolant inférieur formant un premier isolant inférieur (13, 33, 43, 53, 73) ; et
dans lequel l'élément de contact du premier connecteur forme un premier élément de contact (15),
dans lequel le second connecteur comprend :
un second boîtier (22) qui est configuré pour être fixé à la pointe du tube de distribution de fluide, est opposé au premier boîtier, et est constitué d'une résine isolante ; et
un second élément de contact (25) qui est conductible, est maintenu par le second boîtier, et correspond au premier élément de contact,
dans lequel, lorsque l'orifice de connexion de passage (13B, 34B, 44B, 53B, 83B) est formé d'un seul tenant dans le premier boîtier, une partie de logement d'orifice de connexion de passage (23C, 73C) de forme évidée est formée d'un seul tenant dans le second boîtier, la partie de logement d'orifice de connexion de passage s'étendant le long de la direction de montage et étant configurée pour loger l'orifice de connexion de passage, et
dans lequel, en variante, lorsque la partie de logement d'orifice de connexion de passage (23C, 73C) est formée d'un seul tenant dans le premier boîtier, un orifice de connexion de passage (13B, 34B, 44B, 53B, 83B) de forme tubulaire est formé d'un seul tenant dans le second boîtier, l'orifice de connexion de passage s'étendant le long de la direction de montage et comprenant dans son intérieur un passage de liaison (13C, 34C, 44C, 53C, 83C) s'étendant le long de la direction de montage, et
lorsque le second connecteur est monté avec le premier connecteur, le premier élément de contact (15) est électriquement connecté au second élément de contact (25), et l'orifice de connexion de passage est logé dans la partie de logement d'orifice de connexion de passage moyennant quoi le premier orifice de distribution (R1) communique avec le tube de distribution de fluide (P1, P3) via le passage de liaison (13C, 34C, 44C, 53C, 83C),
dans lequel la partie de logement de connecteur du premier isolant supérieur forme une seconde partie de logement de connecteur (14A, 34A, 44A) pour loger une partie du second connecteur lorsque le premier connecteur est monté sur le second connecteur, et le premier isolant inférieur étant joint au premier isolant supérieur, et
l'objet de montage est maintenu entre le premier isolant supérieur et le premier isolant inférieur, moyennant quoi le premier connecteur (11, 31, 41, 51, 71) est monté sur l'objet de montage.

3. Ensemble connecteur selon la revendication 2,
dans lequel l'orifice de connexion de passage (13B, 34B, 44B, 53B) est formé dans le premier boîtier (12), et
la partie de logement d'orifice de connexion de passage (23C) est formée dans le second boîtier (22).

4. Ensemble connecteur selon la revendication 3, dans lequel l'orifice de connexion de passage (13B, 53B) est formé dans le premier isolant inférieur (13, 53).

5. Ensemble connecteur selon la revendication 3, dans lequel l'orifice de connexion de passage (34B, 44B) est formé dans le premier isolant supérieur (34, 44).

6. Ensemble connecteur selon l'une quelconque des revendications 3 à 5,
dans lequel le passage de liaison (13C, 34C, 44C) pénètre dans le premier boîtier (12) dans la direction de montage, et
le premier orifice de distribution (R1) est disposé au niveau d'une partie d'extrémité du passage de liaison.

7. Ensemble connecteur selon la revendication 3 ou 4, dans lequel le premier boîtier (12) comprend un premier passage (53G) s'étendant dans une direction orthogonale à la direction de montage et faisant communiquer le passage de liaison et le premier orifice de distribution l'un avec l'autre.

8. Ensemble connecteur selon l'une quelconque des revendications 3 à 7, dans lequel la pointe du tube de distribution de fluide (P1, P3) est fixée dans la partie de logement d'orifice de connexion de passage (23C) du second boîtier (22).

9. Ensemble connecteur selon la revendication 8, dans lequel la pointe du tube de distribution de fluide (P1) est fixée dans la partie de logement d'orifice de connexion de passage (23C) tout en étant pliée vers la direction de montage à partir d'une direction orthogonale à la direction de montage.

10. Ensemble connecteur selon la revendication 3,
dans lequel la pointe du tube de distribution de fluide (P3) est fixée dans le second boîtier (22) tout en s'étendant dans une direction orthogonale à la direction de montage,
le second boîtier (22) comprend un passage plié (63B) avec une extrémité de celui-ci orientée dans la direction orthogonale à la direction de montage et une autre extrémité de celui-ci orientée dans la direction de montage, et
la pointe du tube de distribution de fluide (P3) et la partie de logement d'orifice de connexion de passage (23C) communiquent l'une avec l'autre via le passage plié (63B).

11. Ensemble connecteur selon la revendication 2,
dans lequel l'orifice de connexion de passage (83B) est formé dans le second boîtier (22), et
la partie de logement d'orifice de connexion de passage (73C) est formée dans le premier boîtier (12).

12. Ensemble connecteur selon l'une quelconque des revendications 2 à 11,
dans lequel le premier élément de contact (15) a une partie tubulaire (15A) s'étendant le long de la direction de montage et pourvue dans son intérieur d'une partie évidée (15C),
le premier isolant inférieur (13, 33, 43, 53, 73) comprend une saillie (13E) à insérer dans la partie évidée du premier élément de contact, et
la saillie est montée par pression dans la partie évidée avec la partie de câblage de l'objet de montage maintenue entre elles, moyennant quoi le premier élément de contact (15) est électriquement connecté à la partie de câblage (C3).

13. Ensemble connecteur selon l'une quelconque des revendications 2 à 12, dans lequel le premier connecteur (11, 31, 41, 51, 71) comprend une feuille de renforcement (16) maintenue par le premier boîtier et s'étendant le long d'une surface de l'objet de montage pour atteindre un extérieur d'une partie périphérique externe du premier boîtier.

14. Ensemble connecteur selon l'une quelconque des revendications 2 à 13,
dans lequel un tube de distribution côté objet de montage (P2, P4) est joint au premier orifice de distribution du premier boîtier, et
le tube de distribution côté objet de montage communique avec le tube de distribution de fluide (P1, P3) via le passage de liaison.
